# EUROPEAN PATENT SPECIFICATION

(11) **EP 3 763 299 B1**
(45) Date of publication and mention of the grant of the patent: **27.12.2023**
(21) Application number: 19775579.6
(22) Date of filing: 27.03.2019
(51) Int. Cl.: A61B 17/135, A61B 17/12, A61B 17/132, A61F 13/00, A61F 5/34, A61F 5/01, A61B 17/00, A61B 90/00

(54) **TOURNIQUET INSTRUMENT**
STAUBINDENINSTRUMENT
INSTRUMENT GARROT

(30) Priority: 30.03.2018 JP 2018067413
(43) Date of publication of application: 13.01.2021
(73) Proprietor: TERUMO Kabushiki Kaisha, Tokyo 151-0072 (JP)
(72) Inventor: YAMADA, Tomomi, Fujinomiya-shi, Shizuoka 418-0015 (JP); HIGASHI, Yo, Fujinomiya-shi, Shizuoka 418-0015 (JP)
(74) Representative: Casalonga
(86) International application number: PCT/JP2019/013315
(87) International publication number: WO 2019/189439

(56) References cited:
- WO-A1-2016/163326
- CN-Y- 2 210 628
- CN-Y- 2 732 194
- CN-Y- 201 200 443
- US-A- 5 695 520
- US-A1- 2006 190 026
- US-A1- 2006 190 026
- US-A1- 2012 296 369

## Description

### Technical Field

The present invention relates to a hemostatic device.

### Background Art

As one of catheter procedures, a procedure of puncturing a blood vessel (for example, radial artery) of an arm of a patient to introduce various medical elongated bodies into the blood vessel via a puncture site formed in the blood vessel of the arm of the patient and performing a procedure or a treatment on a lesion site is known (refer to PTL 1 below).

The radial artery extending along an arm of a human body is connected to a palmar artery which makes a detour on the hand side. Accordingly, in recent years, a catheter procedure using a distal transradial intervention (dTRI) in which the palmar artery located in the hand is punctured and treatment is performed through the puncture site, has been attempted.

### Citation List

### Patent Literature

PTL 1: JP-A-2008-119517

Patent document US5695520A discloses a pressure-applying device for relieving distress The device includes a pressure applicator having a plate member and a pressure-applying body attached to and protruding from one of a pair of opposite surfaces of the plate member. The plate member has a central portion with a pair of opposite surfaces facing in opposite directions and a peripheral portion surrounding the central portion and having a pair of first opposite sides respectively connected to opposite ends of the central portion and a pair of second opposite sides spaced from a pair of opposite edges of the central portion so as to define respective elongated enclosed slots within the plate member between the second opposite sides of the peripheral portion and the pair of opposite edges of the central portion of the plate member. The device also includes a flexible strap attached to the plate member and adapted for wrapping and securing around a part of the body of a user so that the pressure-applying body is between the flexible strap and part of the body and is in contact with a selected point on the part of the body of the user for applying pressure thereto.

Patent document US2012296369A1 discloses a compression unit adapted to be arranged around a patient's forearm to provide pressure to a radial artery puncture site, wherein the compression unit comprises a unit attachment band provided with a frame, and a compression element in the shape of an inflatable bladder, the compression element is further adapted to be arranged within said frame, such that when the compression unit is arranged around a patient's forearm, the compression element provides pressure to the puncture wound when the compression element is inflated.

### Summary of Invention

### Technical Problem

A palmar artery is located in the hand which has many movable portions such as fingers. Therefore, after a catheter procedure is performed, when an operator performs hemostasis on a puncture site of the palmar artery located in the hand, it may be difficult to appropriately compress the puncture site by an existing arm or leg hemostatic device. Therefore, there is a demand for a hemostatic device capable of effectively performing compression-hemostasis on the puncture site located in the hand.

Regarding the puncture site of the hand, since the hand has many movable portions such as fingers, compared with the puncture site of the arm or leg, it is difficult to fix the hemostatic device to the puncture site. Accordingly, the hemostatic device for performing the hemostasis on the puncture site of the hand needs to be disposed so that a pressing portion applying a compression force to the puncture site is not displaced from the puncture site. Moreover, when a patient extends or holds a hand, a shape of the hand is easily changed. Therefore, in the hemostatic device for performing the hemostasis on the puncture site of the hand, when the patient moves his or her hand while the hemostasis is performed, a gap is easily generated between the pressing portion and the puncture site. Accordingly, in the hemostatic device for performing the hemostasis on the puncture site of the hand, it is important to appropriately maintain the compression to the puncture site even when the movement of the hand or the like occurs.

The present invention is made in consideration of the above-described problems, and an object thereof is to provide a hemostatic device capable of being easily fixed to a site of a hand where bleeding is to be stopped and maintaining a compression force of a pressing member to the site where the bleeding is to be stopped.

### Solution to Problem

The invention is defined by appended claim 1.

According to an aspect of the present invention, there is provided a hemostatic device including: a covering member configured to be disposed on a finger of a patient to cover a site on a dorsal side of a hand of the patient where bleeding is to be stopped; a pressing member configured to compress the site where the bleeding is to be stopped in a state where the covering member covers the site where the bleeding is to be stopped; and a linear member configured to secure the covering member to the patient, in which the covering member has a hole portion into which the linear member can be inserted, the linear member has a connection portion which is connected to the covering member, the linear member is configured to form a space into which a portion of the linear member can be inserted between the covering member and the linear member to secure the covering member to the hand of the patient in a state where the linear member is inserted into the hole portion, and the pressing member is disposed on a distal side of the covering member relative to a virtual line passing through the hole portion and the connection portion.

### Advantageous Effects of Invention

In the hemostatic device according to the present invention, the pressing member is disposed in a region which is surrounded by a fingertip covered with the covering member, the hole portion, and the connection portion connecting the linear member and the covering member to each other in a state where the hemostatic device is mounted on the patient. Moreover, in the hemostatic device, a force for securing the covering member to the hand acts on the hole portion side and the connection portion side with the fingertip as a base point in a state where the hemostatic device is mounted on the patient. Accordingly, the pressing member can be in close contact with the hand of the patient, and even when the patient moves the hand, it is possible to prevent a compression force of the pressing member to the site where the bleeding is to be stopped from decreasing or to prevent the pressing member from being displaced. Moreover, in the hemostatic device, the covering member is mounted on the finger of the patient. Accordingly, a predetermined range of the hand including the site where the bleeding is to be stopped with the finger as the base point is covered and the puncture site can be pressed. Therefore, the hemostatic device can be used in various patient regardless of a size of the hand due to an individual difference of the patient.

### Brief Description of Drawings

[Fig. 1] Fig. 1 is a plan view when a hemostatic device according to an embodiment is viewed from an outer surface side.
[Fig. 2] Fig. 2 is a plan view when the hemostatic device according to the embodiment is viewed from an inner surface side.
[Fig. 3] Fig. 3 is a perspective view illustrating a state of mounting the hemostatic device according to the embodiment on a hand of a patient.
[Fig. 4] Fig. 4 is a perspective view illustrating the state of mounting the hemostatic device according to the embodiment on the hand of the patient.
[Fig. 5] Fig. 5 is a perspective view illustrating the state of mounting the hemostatic device according to the embodiment on the hand of the patient.
[Fig. 6] Fig. 6 is a perspective view illustrating a state when the hemostatic device according to the embodiment is mounted on the hand of the patient.
[Fig. 7] Fig. 7 is a perspective view schematically illustrating Modification Example 1 of a cavity adjusting mechanism included in a finger sack portion.
[Fig. 8] Fig. 8 is a perspective view schematically illustrating Modification Example 2 of the cavity adjusting mechanism included in the finger sack portion.
[Fig. 9] Fig. 9 is a view illustrating an operating principle of Modification Example 2 of the cavity adjusting mechanism included in the finger sack portion.

### Description of Embodiments

Hereinafter, an embodiment of the present invention and Modification Examples thereof will be described with reference to the accompanying drawings. Note that the following description does not limit the technical scope or the definition of the terms described in the claims. In addition, dimensional ratios in the drawings are exaggerated for convenience of explanation, and may be different from the actual ratios.

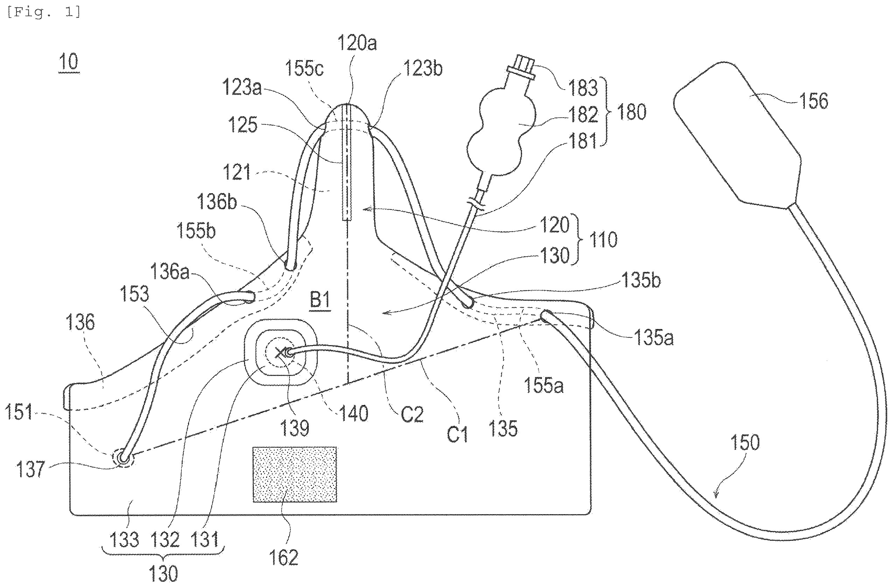

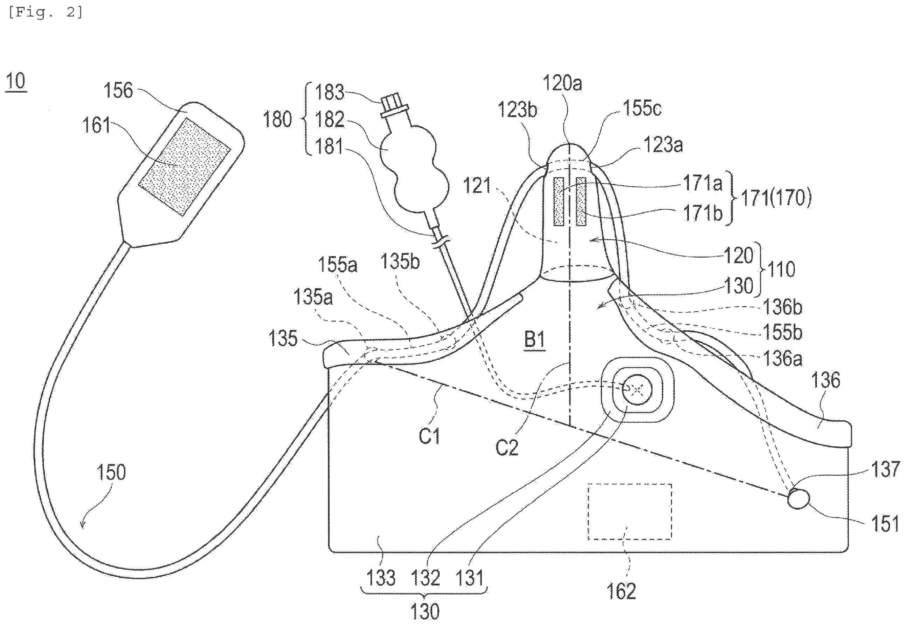

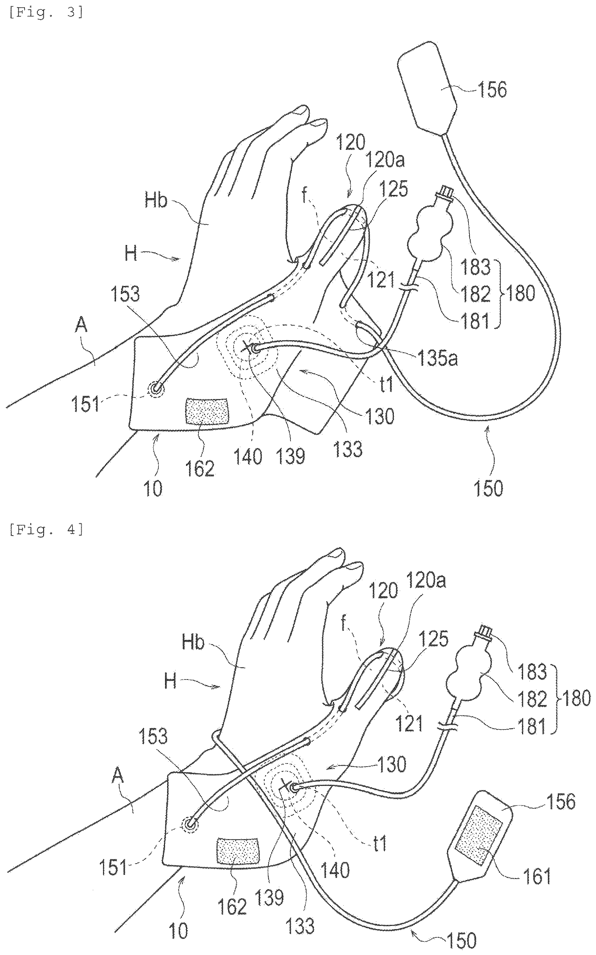

Figs. 1 and 2 are views for explaining a hemostatic device 10 and Figs. 3 to 6 are views for explaining a procedure for mounting the hemostatic device 10.

For example, as illustrated in Figs. 3 to 6, the hemostatic device 10 can be used to perform hemostasis on a puncture site t1 after removing an elongated medical device (for example, introducer) indwelling in the puncture site t1 (corresponding to a "site where bleeding is to be stopped") formed on a radial artery side (for example, a distal radial artery extending around a snuff box or to a fingertip side from the snuff box) of a palmar artery (deep palmar artery) extending to a dorsal side Hb of a hand H of a patient. Note that in the embodiment, an example in which the hemostatic device 10 is used to perform the hemostasis on the puncture site t1 formed in a left hand of the patient is described, but the hand targeted for use of the hemostatic device 10 may be a right hand.

Figs. 1 and 2 illustrate plan views of the hemostatic device 10 in a state before the hemostatic device 10 is mounted on the hand H of the patient. In descriptions of a specification, a surface (mounting surface) on a side of a covering member 110 included in the hemostatic device 10 facing a body surface of the hand H is referred to as an "inner surface" (refer to Fig. 2), and a surface opposite to the inner surface is referred to as an "outer surface" (refer to Fig. 1) .

In the following descriptions of the specification, a distal side of the covering member 110 is a side located on a fingertip side of a thumb f when the hemostatic device 10 is mounted on the hand H of the patient, and a proximal side of the covering member 110 is a side located on a forearm A side (wrist side) when the hemostatic device 10 is mounted on the hand H of the patient. In Figs. 1 and 2, the distal side is a left side in the drawings and the proximal side is a right side in the drawings.

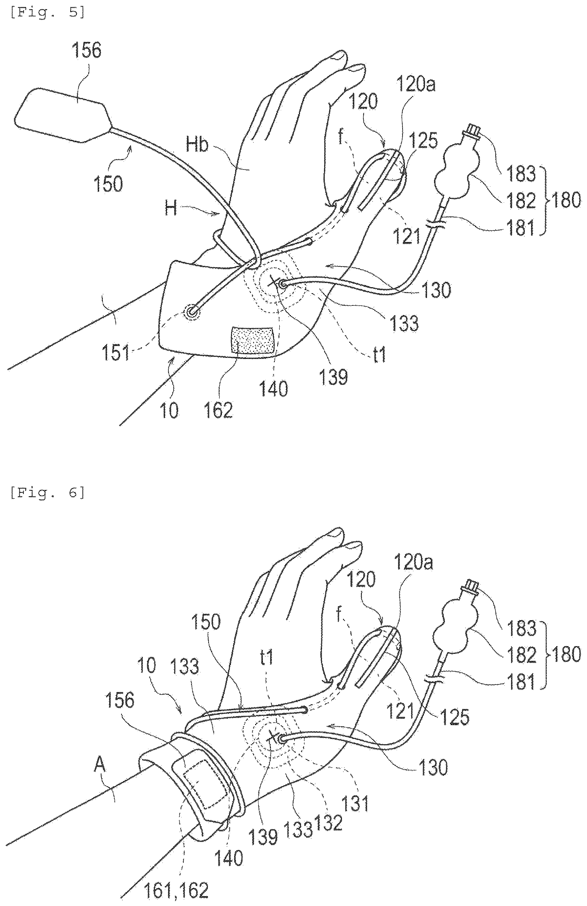

As illustrated in Figs. 1 and 2, the hemostatic device 10 includes the covering member 110 configured to be disposed on the thumb f of the patient to cover the puncture site t1 on the dorsal side Hb of the hand H of the patient (refer to Fig. 6), a pressing member 140 configured to compress the puncture site t1 in a state where the covering member 110 covers the puncture site t1, and a linear member 150 configured to secure the covering member 110 to the patient.

As illustrated in Figs. 1 and 2, the covering member 110 has a first hole portion 135a (corresponding to a "hole portion") into which the linear member 150 can be inserted.

As illustrated in Figs. 1 and 2, the linear member 150 has a connection portion 151 which is connected to the covering member 110. The linear member 150 can form a space 153 into which a portion of the linear member 150 can be inserted between the covering member 110 and the linear member 150 to secure the covering member 110 to the hand H of the patient in a state where the linear member 150 is inserted into the first hole portion 135a.

As illustrated in Figs. 1 and 2, the pressing member 140 is disposed on the distal side of the covering member 110 relative to a virtual line C1 passing through the first hole portion 135a and the connection portion 151. Here, as illustrated in Fig. 1, the virtual line C1 is a straight line which connects the first hole portion 135a and the connection portion 151 to each other in a state where the hemostatic device 10 is not mounted on the hand H of the patient and the covering member 110 is spread. Note that, in Figs. 1 and 2, a virtual line which extends substantially perpendicularly from a tip 120a of the covering member 110 to a proximal side of the hemostatic device 10 and divides the virtual line C1 into two in the state where the hemostatic device 10 is not mounted on the hand H of the patient and the covering member 110 is spread is indicated by a reference sign C2.

Note that the hemostatic device 10 may be configured so that at least a portion of the pressing member 140 is disposed on the distal side of the covering member 110 relative to the virtual line C1 passing through the first hole portion 135a and the connection portion 151 to cause the pressing member 140 to comes into close contact with the hand H of the patient. Accordingly, as illustrated in Fig. 1, the "pressing member 140 being disposed on the distal side of the covering member 110 relative to the virtual line C1 passing through the first hole portion 135a and the connection portion 151" may mean that a half or more of a surface area of the pressing member is disposed on the distal side of the covering member 110 relative to the virtual line C1 passing through the first hole portion 135a and the connection portion 151 in the state where the hemostatic device 10 is not mounted on the hand H of the patient and the covering member 110 is spread.

In the hemostatic device 10, the pressing member 140 is disposed in a region B1 which is surrounded by the fingertip of the thumb f covered with the covering member 110, the first hole portion 135a, and the connection portion 151 in the state where the hemostatic device 10 is mounted on the patient. Note that, as illustrated in Figs. 1 and 2, the virtual line C1 is a substantially straight line in the state where the hemostatic device 10 is spread, but as illustrated in Fig. 6, the virtual line C1 (not illustrated) is arcuate along an outer peripheral surface of the hand H of the patient in the state where the hemostatic device 10 is mounted on the hand H of the patient. In the present embodiment, the pressing member 140 is disposed in the region B1 regardless of whether the virtual line C1 extends linearly or arcuately.

As illustrated in Fig. 1, Fig. 2, and Fig. 6, the covering member 110 includes a finger sack portion 120 configured to be disposed in the thumb f of the patient, and a covering portion 130 which is formed on a proximal side of the finger sack portion 120 and configured to cover the dorsal side Hb of the hand H of the patient and the puncture site t1.

The finger sack portion 120 includes a lumen 121 into which the thumb f can be inserted, and a first through hole (corresponding to "through-hole") 123a and a second through hole (corresponding to "through-hole") 123b through which the linear member 150 passes.

The hemostatic device 10 includes one finger sack portion 120 into which the thumb f is inserted. However, for example, in the hemostatic device 10, the finger sack portion 120 may be configured so that a finger (any one of a little finger, a ring finger, a middle finger, and a forefinger) other than the thumb f is inserted into the finger sack portion 120, or may be configured so that a plurality of fingers are inserted into one finger sack portion 120. Moreover, the hemostatic device 10 may include a plurality of finger sack portions into which any one finger or a plurality of fingers can be inserted.

As illustrated in Fig. 2, the finger sack portion 120 includes a cavity adjusting mechanism 170 which can adjust a size of the lumen 121 of the finger sack portion 120.

The cavity adjusting mechanism 170 includes a holding member 171 which is disposed on the outer surface of the finger sack portion 120 and can hold the finger sack portion in a state where the lumen 121 of the finger sack portion 120 is narrowed. In the present embodiment, the cavity adjusting mechanism 170 includes a pair of surface fasteners 171a and 171b which is disposed on the outer surface of the finger sack portion 120. For example, the surface fastener 171a can be configured on a female side (or male side) of the surface fastener, and for example, the surface fastener 171b can be configured on the male side (or female side) of the surface fastener. The surface fastener 171a and the surface fastener 171b are secured to each other to face each other, and thus, the cavity adjusting mechanism 170 can adjust the lumen 121 of the finger sack portion 120 to be narrowed. Moreover, the securing between the surface fastener 171a and the surface fastener 171b is released, and thus, the cavity adjusting mechanism 170 can adjust the lumen 121 of the finger sack portion 120 to be widened. Note that a specific configuration of the holding member 171 is not particularly limited as long as the holding member 171 can maintain a narrowed state of the lumen 121 of the finger sack portion 120, and can be configured of a belt-shaped fixing member (securing band) or the like, for example.

As illustrated in Fig. 1, the finger sack portion 120 has a guide marker 125 which provides a guide for maintaining a positional relationship between the finger sack portion 120 and the pressing member 140 when the covering member 110 is mounted on the hand H of the patient.

The guide marker 125 extends substantially linearly along an extending direction of the finger sack portion 120 from the tip 120a of the finger sack portion 120 in the state where the hemostatic device 10 is not mounted to the hand H of the patient. As illustrated in Fig. 1, the guide marker 125 is formed on an outer surface side of the finger sack portion 120. An operator such as a doctor confirms a shape (extended shape) of the guide marker 125 when the hemostatic device 10 is mounted on the hand H of the patient (refer to Figs. 5 and 6) . For example, in a case where the guide marker 125 is twisted when the finger sack portion 120 is disposed in the thumb f of the patient, the operator can check that the finger sack portion 120 is twisted and disposed with respect to the thumb f. For example, if the finger sack portion 120 is twisted and disposed with respect to the thumb f, when the covering member 110 is mounted on the hand H of the patient, it is difficult to correctly position the pressing member 140 attached to the covering member 110 at the puncture site t1. Accordingly, in a case where the guide marker 125 is twisted and disposed, the operator adjusts the shape of the finger sack portion 120 so that the guide marker 125 has a substantially linear shape, and thus, the finger sack portion 120 can be appropriately disposed with respect to the thumb f.

Note that, as illustrated in Fig. 1, the guide marker 125 is disposed so as not to overlap the pressing member 140 on an extension line along which the guide marker 125 extends to the proximal side in the state where the hemostatic device 10 is not mounted on the hand.

A color, a thickness, and a design (for example, a continuous straight line, a broken line, or the like) of the guide marker 125 are not particularly limited and may be arbitrary.

As illustrated in Figs. 1 and 2, the covering portion 130 includes a first region 131, a second region 132 which is disposed around the first region 131 and is formed of a member having elasticity higher than a material forming the first region 131, and a third region 133 which is disposed around the second region 132 and is formed of a material having elasticity lower than a material forming the second region 132.

The third region 133 of the covering portion 130 is a portion which is formed to have the largest area in the covering portion 130 and constitutes a main body portion of the covering portion 130. As illustrated in Fig. 6, in the state where the hemostatic device 10 is mounted on the hand H of the patient, the third region 133 is disposed to cover a portion of the dorsal side Hb of the hand H of the patient, a portion of a palm of the hand H of the patient, a wrist of the patient, and a portion of the forearm A of the patient.

The third region 133 of the covering portion 130 has a first insertion portion 135 and a second insertion portion 136 through which the linear member 150 is inserted. The first insertion portion 135 and the second insertion portion 136 are formed on the inner surface side of the covering portion 130 (refer to Fig. 2).

Moreover, the third region 133 of the covering portion 130 has the first hole portion 135a and a second hole portion 135b which communicate with the first insertion portion 135 from the outer surface side of the covering member 110, a third hole portion 136a and a fourth hole portion 136b which communicate with the second insertion portion 136 from an outer surface 110b side of the covering member 110, and a connection hole portion 137 which connects the linear member 150 to the covering portion 130.

As illustrated in Figs. 1 and 2, the first insertion portion 135 is formed on the inner surface side of the covering portion 130. The first insertion portion 135 has a space into which a portion 155a of the linear member 150 is inserted. In addition, the second insertion portion 136 is formed on the inner surface side of the covering portion 130. The second insertion portion 136 has a space into which a portion 155b of the linear member 150 is inserted.

As illustrated in Figs. 1 and 2, the first insertion portion 135 and the second insertion portion 136 are disposed in pairs in the vicinity of each end portion in a width direction (a right-left direction in Figs. 1 and 2) of the covering member 110 in the state where the covering member 110 spreads. Note that a specific position, a shape, a size, or the like of each of the insertion portions 135 and 136 is not particularly limited. A specific position, a shape, a size, or the like of each of the hole portions 135a, 135b, 136a, and 136b is not particularly limited.

The pressing member 140 is constituted by a balloon which can be inflated and deflated. An injection portion 180 for inflating or deflating the pressing member 140 is connected to the pressing member 140.

The injection portion 180 includes a tube 181 which communicates with an internal space (not illustrated) of the pressing member 140 and has flexibility, a bag body 182 which is disposed in one end portion of the tube 181 to communicate with a lumen of the tube 181, and a tubular connector 183 which houses a check valve (not illustrated) connected to the bag body 182. When the operator or the like inflates the pressing member 140, the operator inserts a tip tube portion of a syringe (not illustrated) into the connector 183 to open the check valve and pushes a plunger of the syringe to inject air in the syringe to the internal space of the pressing member 140. When the pressing member 140 is inflated by the above-described operation, the bag body 182 which communicates with the internal space of the pressing member 140 via the tube 181 is inflated. The operator checks the inflation of the bag body 182, and thus, the operator can easily check visually that the pressing member 140 can be pressurized without air leakage. Note that a fluid used to inflate the pressing member 140 is not limited to air. When the operator deflates the pressing member 140 (or when the operator decreases the compression force of the pressing member 140), the operator connects the syringe to the connector 183. The operator operates the syringe to discharge the air of the internal space of the pressing member 140, and thus, the operator can deflate the pressing member 140.

Moreover, the pressing member 140 is not limited to the balloon as long as the pressing member 140 can apply the compression force to the puncture site t1. For example, as the pressing member 140, it is possible to use a mechanical member in which an amount of pushing on the hand H is variable using an external operation such as rotation, a member configured to include a plastic resin material or gel for pushing the hand H so as to provide a surface pressure, a member including hydrophilic gel or a wound material (dressing material) to be brought into contact with the puncture site t1, a member including gel which gradually reduces the compression force by decreasing water content with the lapse of time, an elastic material such as sponge-like substances, aggregates of fibers such as cotton (padding), metal, a member having a predetermined three-dimensional shape(spherical, ellipsoidal, or triangular pyramid shape), or a member obtained by appropriately combining these materials with each other.

The pressing member 140 is disposed at a position (a position where a portion of the pressing member 140 or the entire pressing member 140 overlaps the first region 131 in the plan views illustrated in Figs. 1 and 2) corresponding to the first region 131 of the covering portion 130. In addition, the pressing member 140 is arranged on the inner surface side of the first region 131 facing the hand H of the patient when the hemostatic device 10 is mounted on the hand H of the patient (refer to Fig. 2). The pressing member 140 can be secured to the inner surface of the first region 131 by a known method such as adhesion or fusion.

The first region 131 has a marker portion 139 for positioning the pressing member 140 so that the pressing member 140 overlaps the puncture site t1 when the hemostatic device 10 is mounted on the hand H of the patient. The marker portion 139 is disposed at a substantially central position of the pressing member 140 in a plan view.

A specific shape and color of the marker portion 139, a method for forming the marker portion 139 in the first region 131, or the like is not particularly limited. Note that, preferably, portions of the pressing member 140 and the first region 131 overlapping the marker portion 139 in a plan view and peripheries of the portions are translucent or colored and transparent. Accordingly, the operator can visually recognize the puncture site t1 from the outer surface side of the marker portion 139 even when the marker portion 139 overlaps the puncture site t1.

The second region 132 is disposed to surround a periphery of the first region 131. Note that a range (area in a plan view) or a shape in which the first region 131 is formed on the covering portion 130, a range (area in a plan view) or a shape in which the second region 132 is formed on the covering portion 130, or the like is not limited to the illustrated range or shape. Moreover, a specific shape, a size, or the like of the third region 133 is not particularly limited.

For example, the pressing member (balloon) 140 can be formed of polyolefin such as polyvinyl chloride, polyethylene, polypropylene, polybutadiene, and ethylene-vinyl acetate copolymer (EVA), polyester such as polyethylene terephthalate (PET) and polybutylene terephthalate (PBT), various thermoplastic elastomers such as polyvinylidene chloride, silicone, polyurethane, polyamide elastomer, polyurethane elastomer, and polyester elastomer, or any optional combination thereof (blend resin, polymer alloy, laminate, or the like).

Moreover, for example, the first region 131 of the covering portion 130 can be formed of an acrylic resin, polyvinyl chloride (particularly, hard vinyl chloride), polyolefin such as polyethylene, polypropylene and polybutadiene, polyester such as polyethylene terephthalate (PET) and polybutylene terephthalate (PBT), polystyrene, poly-(4-methylpentene-1), polycarbonate, an ABS resin, polymethylmethacrylate, polyacetal, polyacrylate, polyacrylonitrile, ionomer, acrylonitrile-butadiene-styrene copolymer, polyvinylidene fluoride, a fluorine-based resin such as polytetrafluoroethylene, a butadiene-styrene copolymer, an aromatic or aliphatic polyamide, or the like. Note that, preferably, the first region 131 is formed of a material having elasticity lower than those of the second region 132 and the third region 133. Moreover, it is preferable that the first region 131 is substantially transparent. Accordingly, the operator can visually recognize the puncture site t1 of the patient through the first region 131 from the outside.

In addition, for example, the second region 132 of the covering portion 130 can be formed of the same material as that of the pressing member (balloon) 140, that is, polyolefin such as polyvinyl chloride, polyethylene, polypropylene, polybutadiene, and ethylene-vinyl acetate copolymer (EVA), polyester such as polyethylene terephthalate (PET) and polybutylene terephthalate (PBT), various thermoplastic elastomers such as polyvinylidene chloride, silicone, polyurethane, polyamide elastomer, polyurethane elastomer, and polyester elastomer, or any optional combination thereof (blend resin, polymer alloy, laminate, or the like). Note that, preferably, the second region 132 is formed of a material having elasticity higher than those of the first region 131 and the third region 133.

Moreover, for example, the third region 133 of the covering portion 130 can be formed of the same material as that of the pressing member (balloon) 140, that is, polyolefin such as polyvinyl chloride, polyethylene, polypropylene, polybutadiene, and ethylene-vinyl acetate copolymer (EVA), polyester such as polyethylene terephthalate (PET) and polybutylene terephthalate (PBT), various thermoplastic elastomers such as polyvinylidene chloride, silicone, polyurethane, polyamide elastomer, polyurethane elastomer, and polyester elastomer, or any optional combination thereof (blend resin, polymer alloy, laminate, or the like). For example, the material of the third region 133 may be formed by producing fibers from polyethylene terephthalate (PET) and weaving the fibers. Note that, preferably, the third region 133 is formed of a material having elasticity higher than that of the first region 131 and lower than that of the second region 132. Moreover, the material of third region 133 is not limited to the resin material and may use a paper material or a leather material. Note that, for example, the finger sack portion 120 can be formed integrally with the third region 133 using the same material as that of the third region 133 of the covering portion 130.

Moreover, preferably, in the present embodiment, the first region 131 is formed of a material having elasticity lower than those of the second region 132 and the third region 133. Moreover, preferably, the second region 132 is formed of a material having elasticity higher than that of the third region 133. As an example of a combination of the above-described materials, the first region 131 can be formed of a hard vinyl chloride material, the second region 132 can be formed of a polyurethane material, and the third region 133 can be formed of a soft vinyl chloride material or a material produced by weaving PET fibers. In addition, preferably, the first region 131 is formed of a material which is harder than those of the second region 132 and the third region 133. Note that the first region 131 may be configured to be harder than the second region 132 and the third region 133 by adjusting thicknesses of the second region 132 and the third region 133.

The linear member 150 is constituted by a string-shaped member having a predetermined length and a predetermined outer diameter. A specific length and sectional shape of the linear member 150, the material forming the linear member 150, and the like are not particularly limited.

As illustrated in Fig. 2, the connection portion 151 which connects the linear member 150 to the covering portion 130 is provided on one end portion of the linear member 150. The one end portion of the linear member 150 is inserted from the outer surface side of the covering member 110 into the connection hole portion 137 and is disposed on the inner surface side of the covering member 110.

The one end portion of the linear member 150 may come out of the connection hole portion 137, and thus, the connection portion 151 is configured to prevent disconnection of the linear member 150 and the covering member 110. In the present embodiment, the connection portion 151 is constituted by a locking member having an outer shape larger than the connection hole portion 137. The connection portion 151 is not secured to the covering member 110. Therefore, the linear member 150 can prevent the linear member 150 from coming out of the connection hole portion 137, and when the linear member 150 is wound around the hand H of the patient, the linear member 150 can be moved according to the shape of the hand H.

An interlock portion 156 for interlocking the linear member 150 to the outer surface of the covering portion 130 when the hemostatic device 10 is mounted on the hand H of the patient is provided on the other end portion (an end portion opposite to the end portion on which the connection portion 151 is provided) of the linear member 150. As illustrated in Fig. 2, a first connecting member 161 is disposed on the interlock portion 156. Further, as illustrated in Fig. 1, a second connecting member 162 which can be interlocked to or separated from the first connecting member 161 is disposed on the outer surface of the third region 133 of the covering portion 130. As illustrated in Fig. 6, when the hemostatic device 10 is mounted on the hand H of the patient, the linear member 150 and the third region 133 of the covering portion 130 are interlocked to each other by the first connecting member 161 and the second connecting member 162. For example, the first connecting member 161 can be configured on a female side (or male side) of a surface fastener, and for example, the second connecting member 162 can be configured on the male side (or female side) of the surface fastener. However, a specific structure of each of the connecting members 161 and 162 is not particularly limited as long as each connecting member has a structure which can be interlocked to or separated from each other.

As illustrated in Figs. 1 and 2, in the linear member 150, the connection portion 151 is disposed on the inner surface side of the covering member 110 in the state where the hemostatic device 10 is not mounted on the hand H of the patient.

In the linear member 150, a predetermined range (a portion having a predetermined length) on the one end portion side provided with the connection portion 151 is inserted into the second insertion portion 136. Specifically, as illustrated in Fig. 1, the linear member 150 is inserted into the second insertion portion 136 from the outer surface side of the covering member 110 through the third hole portion 136a. Moreover, the linear member 150 is led to the outer surface side of the covering member 110 from the second insertion portion 136 through the fourth hole portion 136b.

In the linear member 150, a portion extending between the connection portion 151 of the linear member 150 and the third hole portion 136a forms the space 153 (gap between the outer surface of the covering member 110 and the linear member 150) .

A predetermined range of the linear member 150 which is led to the outside of the second insertion portion 136 through the fourth hole portion 136b is inserted into the lumen 121 of the finger sack portion 120. Specifically, as illustrated in Fig. 1, the linear member 150 is inserted into the lumen 121 of the finger sack portion 120 from the outside of the finger sack portion 120 through the first through hole 123a. Moreover, the linear member 150 is led from the lumen 121 of the finger sack portion 120 to the outside of the finger sack portion 120 through the second through hole 123b.

A predetermined range of the linear member 150 which is led from the lumen 121 of the finger sack portion 120 to the outside through the second through hole 123b is inserted into the first insertion portion 135. Specifically, as illustrated in Fig. 1, the linear member 150 is inserted into the first insertion portion 135 from the outer surface side of the covering member 110 through the second hole portion 135b. In addition, the linear member 150 is led to the outer surface side of the covering member 110 from the first insertion portion 135 through the first hole portion 135a. Note that the interlock portion 156 which is disposed on the other end portion of the linear member 150 prevents the linear member 150 from accidentally coming out through the hole portions 135a and 135b.

Next, an example of using the hemostatic device 10 will be described with reference to Figs. 3 to 6. Hereinafter, a procedure of mounting the hemostatic device 10 on the hand H of the patient will be mainly described.

Fig. 3 illustrates a state when the medical device such as the introducer is inserted into the distal radial artery via the puncture site t1 formed on the dorsal side Hb of the hand H of the patient and various procedures are performed, and thereafter, the hemostasis of the puncture site t1 starts. Note that the medical device is not illustrated in Figs. 3 to 6.

First, as illustrated in Fig. 3, the operator such as a doctor inserts the thumb f into the lumen 121 of the finger sack portion 120. Moreover, the operator disposes the covering member 110 on the hand H and the forearm A of the patient so as to cover the dorsal side Hb of the hand H with the covering portion 130. The operator disposes the marker portion 139 around the puncture site t1 while checking positions of the marker portion 139 and the puncture site t1. Note that, in this case, the operator disposes the guide marker 125 so that the guide marker 125 is located at a center of the thumb f. Accordingly, when the operator disposes the marker portion 139 around the puncture site t1, the operator can check whether the finger sack portion 120 is disposed to be twisted in the thumb f.

Next, as illustrated in Fig. 4, the operator causes the other end portion side on which the interlock portion 156 of the linear member 150 is disposed to pass through the space 153 formed by the linear member 150. Then, the operator disposes the linear member 150 so that the linear member 150 is wound leftward in a circumferential direction of the hand H of the patient.

Next, as illustrated in Fig. 5, the operator folds back the linear member 150 so that a portion of the linear member 150 is hooked on the portion forming the space 153. Then, the operator disposes the linear member 150 so that the linear member 150 is wound rightward in the circumferential direction of the hand H of the patient.

Next, as illustrated in Fig. 6, the operator interlocks the first connecting member 161 disposed on the one end portion of the linear member 150 with the second connecting member 162 disposed on the covering portion 130. Note that the operator checks the positions of the puncture site t1 and the marker portion 139 before interlocking the first connecting member 161 and the second connecting member 162 to each other, and thus, can accurately dispose the pressing member 140 at the puncture site t1.

Next, the operator inflates the pressing member 140 using the injection portion 180 and the syringe (not illustrated) . The pressing member 140 is inflated, and thus, the pressing member 140 applies the compression force to the puncture site t1. The operator removes the medical device such as the introducer from the puncture site t1 while the operator maintains the state in which the pressing member 140 applies the compression force to the puncture site t1.

Effects of the hemostatic device 10 according to the present embodiment will be described.

The hemostatic device 10 according to the present embodiment includes the covering member 110 configured to be disposed on the thumb f of the patient to cover the puncture site t1 on the dorsal side Hb of the hand H of the patient, the pressing member 140 configured to compress the puncture site t1 in the state where the covering member 110 covers the puncture site t1, and the linear member 150 configured to secure the covering member 110 to the patient. The covering member 110 has the first hole portion 135a into which the linear member 150 can be inserted. The linear member 150 has the connection portion 151 which is connected to the covering member 110, and the linear member 150 can form the space 153 into which a portion of the linear member 150 can be inserted between the covering member 110 and the linear member 150 to secure the covering member 110 to the hand H of the patient in a state where the linear member 150 is inserted into the first hole portion 135a. Then, the pressing member 140 is disposed on the distal side of the covering member 110 relative to the virtual line C1 passing through the first hole portion 135a and the connection portion 151.

In the hemostatic device 10 having the above-described configuration, a force for securing the covering member 110 to the hand H acts on the first hole portion 135a side and the connection portion 151 side with the fingertip of the thumb f as a base point in a state where the pressing member 140 applies the compression force to the puncture site t1. Accordingly, the pressing member 140 can be in close contact with the hand H of the patient. Moreover, in the hemostatic device 10, the covering member 110 is mounted on the thumb f of the hand H of the patient. Accordingly, a predetermined range of the hand H including the puncture site t1 with the thumb f as the base point is covered and the puncture site t1 can be pressed. Therefore, the hemostatic device 10 can be used in various patients regardless of a size of the hand H due to an individual difference of the patient.

Moreover, in the hemostatic device 10, the linear member 150 is disposed to be wound along the circumferential direction of the hand H of the patient, and thus, the covering member 110 disposed between the hand H of the patient and the linear member 150 is secured to the hand H of the patient. Accordingly, in the hemostatic device 10, the entire covering member 110 is not secured to the hand H of the patient in the state where the hemostatic device 10 is mounted on the hand H of the patient. Therefore, when the patient moves the hand H, the hemostatic device 10 can prevent a force which restricts the movement from being applied to the hand H of the patient. As a result, the hemostatic device 10 has a high degree of freedom in the movement of the hand H of the patient even while the hemostasis is performed by the hemostatic device 10.

Moreover, the covering member 110 includes the finger sack portion 120 configured to be disposed on the thumb f of the patient and the covering portion 130 which is formed on the proximal side of the finger sack portion 120 and configured to cover the dorsal side Hb and the puncture site t1 of the hand H of the patient. The covering portion 130 has the first region 131, the second region 132 which is disposed around the first region 131 and is formed of the member having elasticity higher than that of the material forming the first region 131, and the third region 133 which is disposed around the second region 132 and is formed of the material having elasticity lower than the material forming the second region 132. The pressing member 140 is disposed at a position corresponding to the first region 131.

In the hemostatic device 10 having the above-described configuration, the finger sack portion 120 is disposed on the thumb f of the patient, and thus, the covering member 110 can be easily mounted on the thumb f. Moreover, the elasticity of the first region 131 of the covering portion 130 is lower than that of the second region 132, and thus, it is possible to prevent a force for pressing the pressing member 140 to the puncture site t1 from being dispersed. In addition, the elasticity of the second region 132 of the covering portion 130 is higher than the elasticity of the first region 131. Accordingly, the second region 132 is in close contact with the hand H around the first region 131, and it is possible to prevent the pressing member 140 from being displaced from the puncture site t1. Moreover, the elasticity of the third region 133 of the covering portion 130 is lower than that of the second region 132. Accordingly, it is possible to prevent the third region 133 from being unintentionally expanded and contracted in the state where the hemostatic device 10 is mounted on the hand H of the patient. Therefore, the hemostatic device 10 can prevent the mounting position of the hemostatic device 10 from being displaced.

Moreover, the material forming the first region 131 of the covering portion 130 has elasticity lower than that of the material forming the third region 133 of the covering portion 130 and is harder than those of the second region 132 and the third region 133. Moreover, in the hemostatic device 10, it is possible to more appropriately maintain the state where the pressing member 140 disposed at the position corresponding to the first region 131 is pressed against the puncture site t1, and thus, hemostatic effects can be enhanced.

Moreover, the finger sack portion 120 includes the cavity adjusting mechanism 170 which can adjust the size of the lumen 121 of the finger sack portion 120. Accordingly, in the hemostatic device 10, it is possible to improve adhesiveness of the finger sack portion 120 to a base or the like of the thumb f by reducing the lumen 121 of the finger sack portion 120. As a result, in the hemostatic device 10, it is possible to prevent the finger sack portion 120 from being displaced.

Moreover, the cavity adjusting mechanism 170 has the holding member 171 which is disposed on the outer surface of the finger sack portion 120 and can hold the finger sack portion 120 in the state where the lumen 121 of the finger sack portion 120 is narrowed. Therefore, in the hemostatic device 10, it is possible to easily maintain the state where the lumen 121 of the finger sack portion 120 is narrowed by using the holding member 171.

Moreover, the finger sack portion 120 has the through holes 123a and 123b through which the linear member 150 can pass. Accordingly, in the hemostatic device 10, the linear member 150 which is disposed at three locations of the first hole portion 135a, the connection portion 151, and the finger sack portion 120 applies a securing force to the first hole portion 135a side and the connection portion 151 side with the first sack portion 120 side as a base point, in the state where the hemostatic device 10 is mounted on the hand H of the patient. Accordingly, in the hemostatic device 10, it is possible to more stably maintain a state where the hemostatic device 10 is secured to the hand H of the patient.

Moreover, the finger sack portion 120 has the guide marker 125 which provides the guide for maintaining the positional relationship between the finger sack portion 120 and the pressing member 140 when the covering member 110 is mounted on the hand H of the patient. Accordingly, in the hemostatic device 10, it is possible to prevent the finger sack portion 120 from being mounted on the thumb f in the state where the finger sack portion 120 is twisted or the like.

Next, Modification Examples of the cavity adjusting mechanism for adjusting the size of the lumen 121 of the finger sack portion 120 will be described. Note that, in descriptions of Modification Examples, the same reference signs are assigned to the same configurations as those of the above-described embodiment, and descriptions thereof are omitted.

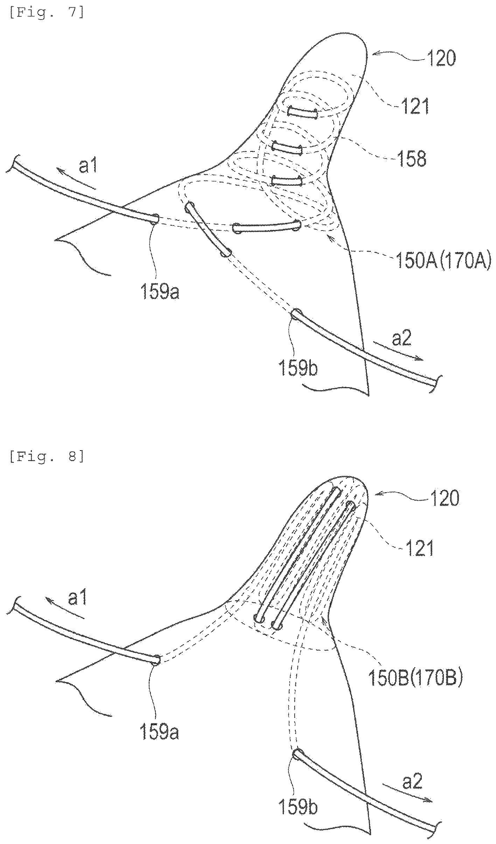

Fig. 7 illustrates a cavity adjusting mechanism 170A according to Modification Example 1.

The cavity adjusting mechanism 170A is constituted by a portion of a linear member 150A. Specifically, the linear member 150A has a winding portion 158 which is disposed so as to be wound in a predetermined shape on the lumen 121 of the finger sack portion 120 and the outer surface side of the finger sack portion 120.

The linear member 150A is led to the outer surface side of the covering portion 130 through each of through holes 159a and 159b. In the linear member 150A, a portion of the winding portion 158 extends spirally on the lumen 121 of the finger sack portion 120 and the outer surface of the finger sack portion 120. The operator pulls both end portion sides of the linear member 150A outside the finger sack portion 120 as illustrated by arrows a1 and a2, and thus, can contract the spirally extending portion of the winding portion 158. The winding portion 158 of the linear member 150A is disposed so that a portion of the winding portion 158 is exposed on the outer surface side of the finger sack portion 120. Accordingly, when the linear member 150A is pulled and deformed so that the winding portion 158 is contracted, the linear member 150A contracts the finger sack portion 120 from the outer surface side of the finger sack portion 120. Therefore, the finger sack portion 120 is deformed so that the lumen 121 of the finger sack portion 120 is narrowed.

Fig. 8 illustrates a cavity adjusting mechanism 170B according to Modification Example 2.

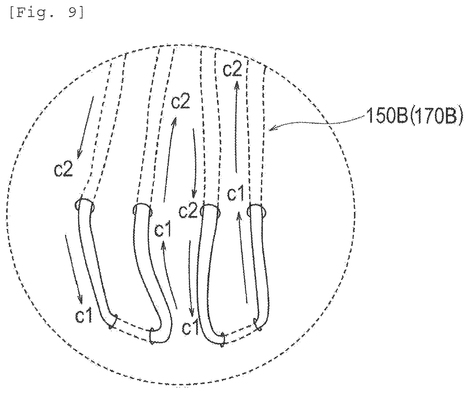

The cavity adjusting mechanism 170B is constituted by a portion of a linear member 150B. A portion of the linear member 150B is disposed in the lumen 121 of the finger sack portion 120, and a portion of the linear member 150B is lead to the outer surface side of the finger sack portion 120. As illustrated in Fig. 9, the linear member 150B is disposed so as to reciprocate along a longitudinal direction (direction of arrow c1) and a depth direction (direction of arrow c2) of the finger sack portion 120. When the operator pulls the linear member 150A as illustrated by the arrows a1 and a2 in Fig. 8 outside the finger sack portion 120, the linear member 150B is pulled to alternately move in the longitudinal direction and the depth direction as illustrated in Fig. 9. Then, in the linear member 150B, a portion (a portion exposed from the finger sack portion 120) disposed near the base portion of the finger sack portion 120 is compressed so as to tighten the finger sack portion 120. Therefore, the operator can easily narrow the lumen 121 of the finger sack portion 120 by pulling the linear member 150B.

Hereinbefore, the hemostatic device according to the present invention is described through the embodiment. However, the present invention is not limited to each configuration described in the specification, and may be appropriately changed based on descriptions of claims.

For example, in the descriptions of the embodiment, the hemostatic device for performing the hemostasis on the puncture site formed in the left hand is exemplified. However, the hemostatic device can be used for performing hemostasis on a puncture site formed in the right hand. When the hemostatic device is used in the right hand, the shape of the covering member, the position of the pressing member, or the like can be appropriately changed to perform the hemostasis on the puncture site formed in the right hand.

Priority is claimed on Japanese Patent Application No. 2018-067413, filed March 30, 2018.

### Reference Signs List

- 10: hemostatic device
- 110: covering member
- 120: finger sack portion
- 120a: tip of covering member
- 121: lumen of covering member
- 123a: first through hole (through hole)
- 123b: second through hole (through hole)
- 125: guide marker
- 130: covering portion
- 131: first region
- 132: second region
- 133: third region
- 135: first insertion portion
- 135a: first hole portion (hole portion)
- 135b: second hole portion
- 136: second insertion portion
- 136a: third hole portion
- 136b: fourth hole portion
- 137: connection hole portion
- 139: marker portion
- 140: pressing member
- 150, 150A, 150B: linear member
- 151: connection portion
- 153: space
- 170, 170A, 170B: cavity adjusting mechanism
- 171: holding member
- 180: injection portion
- A: forearm
- H: hand
- Hb: dorsal side
- f: thumb (finger)
- t1: puncture site (site where bleeding is to be stopped)
- C1: virtual line

## Claims

1. A hemostatic device (10) comprising:
a covering member (110) configured to be disposed on a finger of a patient to cover a site (t1) on a dorsal side (Hb) of a hand (H) of the patient where bleeding is to be stopped;
a pressing member (140) configured to compress the site (t1) where the bleeding is to be stopped in a state where the covering member (110) covers the site (t1) where the bleeding is to be stopped; and
a linear member (150, 150A, 150B) configured to secure the covering member (110) to the patient and constituted by a string-shaped member having a predetermined length and a predetermined outer diameter,
wherein the covering member (110) has a hole portion (135a) into which the linear member (150, 150A, 150B) is inserted,
wherein the linear member (150, 150A, 150B) has a connection portion (151) which is connected to the covering member (110),
wherein the linear member (150, 150A, 150B) is configured to form a space (153) into which a portion of the linear member (150, 150A, 150B) can be inserted between the covering member (110) and the linear member (150, 150A, 150B) to secure the covering member (110) to the hand (H) of the patient in a state where the linear member (150, 150A, 150B) is inserted into the hole portion (135a), and
wherein the pressing member (140) is disposed on a distal side of the covering member (110) relative to a virtual line (C1) passing through the hole portion (135a) and the connection portion (151).

2. The hemostatic device (10) according to claim 1,
wherein the covering member (110) includes a finger sack portion (120) configured to be disposed on the finger of the patient and a covering portion (130) which is formed on a proximal side of the finger sack portion (120) and configured to cover the dorsal side (Hb) of the hand (H) of the patient and the site (t1) where the bleeding is to be stopped,
wherein the covering portion (130) has a first region (131), a second region (132) which is disposed around the first region (131) and is formed of a member having elasticity higher than that of a material forming the first region (131), and a third region (133) which is disposed around the second region (132) and is formed of a material having elasticity lower than a material forming the second region (132), and
wherein the pressing member (140) is disposed at a position corresponding to the first region (131).

3. The hemostatic device (10) according to claim 2,
wherein the material forming the first region (131) has elasticity lower than that of the material forming the third region (133) and is harder than those of the second region (132) and the third region (133).

4. The hemostatic device (10) according to claim 2 or 3,
wherein the finger sack portion (120) includes a cavity adjusting mechanism (170, 170A, 170B) which adjusts a size of a lumen of the finger sack portion (120).

5. The hemostatic device (10) according to claim 4,
wherein the cavity adjusting mechanism (170, 170A, 170B) has a holding member (171) which is disposed on an outer surface of the finger sack portion (120) and holds the finger sack portion (120) in a state where the lumen of the finger sack portion (120) is narrowed.

6. The hemostatic device (10) according to any one of claims 2 to 5,
wherein the finger sack portion (120) has a through hole (123a, 123b) through which the linear member (150, 150A, 150B) passes.

7. The hemostatic device (10) according to any one of claims 2 to 6,
wherein the finger sack portion (120) has a guide marker (125) which provides a guide for maintaining a positional relationship between the finger sack portion (120) and the pressing member (140) when the covering member (110) is mounted on the hand (H) of the patient.

## Patentansprüche

1. Hämostatische Vorrichtung (10), umfassend:
ein Abdeckelement (110), das so konfiguriert ist, dass es an einem Finger eines Patienten angeordnet werden kann, um eine Stelle (t1) auf einer dorsalen Seite (Hb) einer Hand (H) des Patienten abzudecken, an der die Blutung gestoppt werden soll;
ein Druckelement (140), das so konfiguriert ist, dass es die Stelle (t1), an der die Blutung gestoppt werden soll, in einem Zustand zusammendrückt, in dem das Abdeckelement (110) die Stelle (t1), an der die Blutung gestoppt werden soll, abdeckt; und
ein lineares Element (150, 150A, 150B), das so konfiguriert ist, dass es das Abdeckelement (110) an dem Patienten befestigt, und das aus einem schnurförmigen Element besteht, das eine vorbestimmte Länge und einen vorbestimmten Außendurchmesser aufweist,
wobei das Abdeckelement (110) einen Lochabschnitt (135a) aufweist, in den das lineare Element (150, 150A, 150B) eingeführt wird,
wobei das lineare Element (150, 150A, 150B) einen Verbindungsabschnitt (151) aufweist, der mit dem Abdeckelement (110) verbunden ist,
wobei das lineare Element (150, 150A, 150B) so konfiguriert ist, dass es einen Raum (153) bildet, in den ein Abschnitt des linearen Elements (150, 150A, 150B) zwischen dem Abdeckelement (110) und dem linearen Element (150, 150A, 150B) eingeführt werden kann, um das Abdeckelement (110) an der Hand (H) des Patienten in einem Zustand zu befestigen, in dem das lineare Element (150, 150A, 150B) in den Lochabschnitt (135a) eingeführt ist, und
wobei das Druckelement (140) an einer distalen Seite des Abdeckelements (110) relativ zu einer virtuellen Linie (C1), die durch den Lochabschnitt (135a) und den Verbindungsabschnitt (151) verläuft, angeordnet ist.

2. Hämostatische Vorrichtung (10) nach Anspruch 1,
wobei das Abdeckelement (110) einen Fingerbeutelabschnitt (120), der so konfiguriert ist, dass er an dem Finger des Patienten angeordnet werden kann, und einen Abdeckabschnitt (130) umfasst, der an einer proximalen Seite des Fingerbeutelabschnitts (120) ausgebildet ist und so konfiguriert ist, dass er die dorsale Seite (Hb) der Hand (H) des Patienten und die Stelle (t1), an der die Blutung gestoppt werden soll, abdeckt,
wobei der Abdeckabschnitt (130) einen ersten Bereich (131), einen zweiten Bereich (132), der um den ersten Bereich (131) herum angeordnet ist und aus einem Element gebildet ist, das eine höhere Elastizität als die eines den ersten Bereich (131) bildenden Materials aufweist, und einen dritten Bereich (133) aufweist, der um den zweiten Bereich (132) herum angeordnet ist und aus einem Material gebildet ist, das eine geringere Elastizität als ein den zweiten Bereich (132) bildendes Material aufweist, und
wobei das Druckelement (140) an einer Position angeordnet ist, die dem ersten Bereich (131) entspricht.

3. Hämostatische Vorrichtung (10) nach Anspruch 2,
wobei das Material, das den ersten Bereich (131) bildet, eine geringere Elastizität aufweist als das Material, das den dritten Bereich (133) bildet, und härter ist als diejenigen des zweiten Bereichs (132) und des dritten Bereichs (133).

4. Hämostatische Vorrichtung (10) nach Anspruch 2 oder 3,
wobei der Fingerbeutelabschnitt (120) einen Hohlraum-Einstellmechanismus (170, 170A, 170B) aufweist, der eine Größe eines Lumens des Fingerbeutelabschnitts (120) einstellt.

5. Hämostatische Vorrichtung (10) nach Anspruch 4,
wobei der Hohlraum-Einstellmechanismus (170, 170A, 170B) ein Halteelement (171) aufweist, das an einer Außenfläche des Fingerbeutelabschnitts (120) angeordnet ist und den Fingerbeutelabschnitt (120) in einem Zustand hält, in dem das Lumen des Fingerbeutelabschnitts (120) verengt ist.

6. Hämostatische Vorrichtung (10) nach einem der Ansprüche 2 bis 5,
wobei der Fingerbeutelabschnitt (120) ein Durchgangsloch (123a, 123b) aufweist, durch welches das lineare Element (150, 150A, 150B) verläuft.

7. Hämostatische Vorrichtung (10) nach einem der Ansprüche 2 bis 6,
wobei der Fingerbeutelabschnitt (120) eine Führungsmarkierung (125) aufweist, die eine Führung zur Aufrechterhaltung einer Positionsbeziehung zwischen dem Fingerbeutelabschnitt (120) und dem Druckelement (140) bereitstellt, wenn das Abdeckelement (110) an der Hand (H) des Patienten angebracht ist.

## Revendications

1. Dispositif hémostatique (10) comprenant :
un élément de couverture (110) configuré pour être disposé sur un doigt d'un patient afin de couvrir un site (t1) sur une face dorsale (Hb) d'une main (H) du patient où le saignement doit être arrêté ;
un élément de pression (140) configuré pour comprimer le site (t1) où le saignement doit être arrêté dans un état où l'élément de couverture (110) couvre le site (t1) où le saignement doit être arrêté ; et
un élément linéaire (150, 150A, 150B) configuré pour fixer l'élément de couverture (110) au patient et constitué d'un élément en forme de corde ayant une longueur et un diamètre extérieur prédéterminés,
dans lequel l'élément de couverture (110) possède une partie trouée (135a) dans laquelle l'élément linéaire (150, 150A, 150B) est inséré,
l'élément linéaire (150, 150A, 150B) possède une partie de connexion (151) qui est reliée à l'élément de couverture (110),
dans lequel l'élément linéaire (150, 150A, 150B) est configuré pour former un espace (153) dans lequel une partie de l'élément linéaire (150, 150A, 150B) peut être insérée entre l'élément de couverture (110) et l'élément linéaire (150, 150A, 150B) pour fixer l'élément de couverture (110) à la main (H) du patient dans un état où l'élément linéaire (150, 150A, 150B) est inséré dans la partie de trou (135a), et
dans lequel l'élément de pression (140) est disposé sur un côté distal de l'élément de couverture (110) par rapport à une ligne virtuelle (C1) passant par la partie trouée (135a) et la partie de connexion (151).

2. Dispositif hémostatique (10) selon la revendication 1,
dans lequel l'élément de couverture (110) comprend une partie de sac de doigt (120) configurée pour être disposée sur le doigt du patient et une partie de couverture (130) qui est formée sur un côté proximal de la partie de sac de doigt (120) et configurée pour couvrir le côté dorsal (Hb) de la main (H) du patient et le site (t1) où le saignement doit être arrêté,
dans lequel la partie de couverture (130) comporte une première région (131), une deuxième région (132) qui est disposée autour de la première région (131) et qui est formée d'un élément ayant une élasticité supérieure à celle d'un matériau formant la première région (131), et une troisième région (133) qui est disposée autour de la deuxième région (132) et qui est formée d'un matériau ayant une élasticité inférieure à celle d'un matériau formant la deuxième région (132), et
dans lequel l'élément de pressage (140) est disposé au niveau d'une position correspondant à la première région (131).

3. Dispositif hémostatique (10) selon la revendication 2,
dans lequel le matériau formant la première région (131) a une élasticité inférieure à celle du matériau formant la troisième région (133) et est plus dur que ceux de la deuxième région (132) et de la troisième région (133) .

4. Dispositif hémostatique (10) selon la revendication 2 ou la revendication 3,
dans lequel la partie de sac de doigt (120) comprend un mécanisme de réglage de la cavité (170, 170A, 170B) qui ajuste la taille d'une lumière de la partie de sac de doigt (120).

5. Dispositif hémostatique (10) selon la revendication 4,
dans lequel le mécanisme de réglage de la cavité (170, 170A, 170B) comporte un élément de maintien (171) disposé sur une surface extérieure de la portion de sac de doigt (120) et qui maintient la portion de sac de doigt (120) dans un état où la lumière de la portion de sac de doigt (120) est rétrécie.

6. Dispositif hémostatique (10) selon l'une quelconque des revendications 2 à 5, dans lequel la partie de sac de doigt (120) comporte un trou de passage (123a, 123b) à travers lequel passe l'élément linéaire (150, 150A, 150B) .

7. Dispositif hémostatique (10) selon l'une quelconque des revendications 2 à 6, dans lequel la partie de sac de doigt (120) comporte un repère de guidage (125) qui fournit un guide pour maintenir une relation de position entre la partie de sac de doigt (120) et l'élément de pression (140) lorsque l'élément de couverture (110) est monté sur la main (H) du patient.
